# EUROPEAN PATENT SPECIFICATION

(11) **EP 3 784 125 B1**
(45) Date of publication and mention of the grant of the patent: **02.10.2024**
(21) Application number: 19726223.1
(22) Date of filing: 22.04.2019
(51) Int. Cl.: A61B 5/022, A61B 5/00

(54) **FINGER CUFF WITH A BUTTERFLY SHAPE FOR MEASURING BLOOD PRESSURE, AND METHOD FOR MEASURING BLOOD PRESSURE USING THE FINGER CUFF**
FINGERMANSCHETTE MIT SCHMETTERLINGSFORM ZUR BLUTDRUCKMESSUNG, UND VERFAHREN ZUR BLUTDRUCKMESSUNG MIT DER FINGERMANSCHETTE
MANCHON DIGITAL DE MESURE DE LA TENSION ARTÉRIELLE AYANT UNE FORME DE PAPILLON, ET PROCÉDÉ DE MESURE DE LA TENSION ARTÉRIELLE UTILISANT LE MANCHON DIGITAL

(30) Priority: 24.04.2018 US 201862662125 P; 25.03.2019 US 201916364018
(43) Date of publication of application: 03.03.2021
(73) Proprietor: Edwards Lifesciences Corporation, Irvine, CA 92614 (US)
(72) Inventor: SETTELS, Jacobus Jozef Gerardus Maria, 1426 AR De Hoef (NL)
(74) Representative: Eisenführ Speiser
(86) International application number: PCT/US2019/028446
(87) International publication number: WO 2019/209675

(56) References cited:
- WO-A1-2004/100783
- WO-A1-2009/014419
- WO-A1-2010/129131
- WO-A1-2019/113100
- WO-A2-2008/079952

## Description

### BACKGROUND

### CROSS-REFERENCE TO RELATED APPLICATIONS

This application claims the benefit of U.S. Provisional Patent Application No. 62/662,125 filed April 24th, 2018.

### Field

The invention relates to non-invasive hemodynamic measurements. More particularly, the invention relates to a finger cuff having a sensor for blood pressure measurements. The invention is defined by the appended claims 1-4.

### Relevant Background

Volume clamping is a technique for non-invasively measuring blood pressure in which pressure is applied to a patient's finger in such a manner that arterial pressure may be balanced by a time varying pressure to maintain a constant arterial volume. In a properly fitted and calibrated system, the applied time varying pressure should be equal to the arterial blood pressure in the finger. The applied time varying pressure may be measured to provide a reading of the patient's arterial blood pressure.

This may be accomplished by a finger cuff that is arranged or wrapped around a finger of a patient. The finger cuff may include an infrared light source, an infrared sensor, and an inflatable bladder. The infrared light may be sent through the finger in which a finger artery is present. The infrared sensor picks up the infrared light and the amount of infrared light registered by the sensor may be inversely proportional to the artery diameter.

In the finger cuff implementation, by inflating the bladder in the finger cuff, a pressure is exerted on the finger artery. If the pressure is high enough, it will compress the artery and the amount of light registered by the sensor will increase. The amount of pressure necessary in the inflatable bladder to compress the artery is dependent on the blood pressure. By controlling the pressure of the inflatable bladder, such that, the diameter of the finger artery is kept constant at its unloaded diameter, the blood pressure may be monitored in very precise detail, as the pressure in the inflatable bladder is directly linked to the blood pressure. In a typical present day finger cuff implementation, a volume clamp system is used with the finger cuff. The volume clamp system typically includes a pressure generating system and a regulating system that includes: a pump, a valve, and a pressure sensor in a closed loop feedback system that are used to clamp the arterial volume as used in the measurement of the arterial pressure. To accurately measure blood pressure, the feedback loop provides sufficient pressure generating and releasing capabilities to match the pressure oscillations of the patient's blood pressure.

Today, finger cuff based blood pressure monitoring devices generally use the same technology (e.g., photoplethysmography or similar technologies) to measure blood pressure. Unfortunately, such finger cuff devices may not be easily attachable to a patient's finger and may not be that accurate due to the finger cuff's positioning on the patient's finger. That is, attaching the finger cuff in a suboptimal way negatively influences the measurement reliability and accuracy of the volume clamp system. Moreover, there is no intrinsic guidance or limit built in present day finger cuffs to ensure that a correctly sized finger cuff is used, thereby reducing the measurement reliability and accuracy of the volume clamp system.

WO 2009/014419 A1 relates to a cuff for determining a physiological parameter, said cuff comprising a photoplethysmograph arranged with an emitter for emitting a radiation in a direction of a tissue to be investigated, a detector for detecting the radiation from the tissue and an inflatable bladder for transferring pressure to the tissue, said inflatable bladder comprising a back-layer and a top-layer, wherein the top-layer is conceived to be brought into contact with the tissue, the top-layer being substantially more flexible than the back-layer. The disclosure further relates to a measurement system.

WO 2004/100783 A1 provides an automatic blood pressure monitor for measuring the arterial blood pressure at a location on a patient. The automatic monitor of the disclosure does not require an external pump of any type. The disclosure uses a propellant supply chamber containing a predetermined quantity of propellant in order to inflate a pressure occluding bladder, thus allowing the measurement to be made. All of the elements comprising the monitor are attached to or are an integral part of a strap, which supports the monitor at the desired location on the patient. The monitor of the disclosure is inexpensive and simple to operate and can be used by non-professional persons in non-clinical or non-laboratory environments.

WO 2019/113100 A1, which was published after the filing date of the present disclosure, relates to a butterfly cuff for monitoring physiological cycles has an exterior and interior surface and securing means. The cuff consists of a body having an upper curve, a lower curve and a height (CH) therebetween. A sensor adhered with a flexible protective overlay to the interior surface is in communication with a microprocessor storage means. The microprocessor storage means can be within the cuff or separate therefrom with communication between the sensor and the storage means being wireless or through a communication member. A locking tab, having a width less than the body, extends from one side of the body. A slot retaining area extends from the body on a side opposite the locking tab and contains a slot dimensioned to receive the locking tab. A pull tab is adjacent the slot retaining area opposite the body. The pull tab shares a base with the slot retaining area and has a top line and an end width less than the width of the body. The cuff can be manufactured from a hook and loop material or have other securing methods to affix the cuff in place.

### SUMMARY OF THE INVENTION

A finger cuff according to the present invention is defined in claim 1, and a pertaining method to measure a patient's blood pressure is defined in claim 3. The dependent claims are directed to embodiments of the finger cuff and of the method.

### BRIEF DESCRIPTION OF THE DRAWINGS

FIG. 1 is a diagram of an optional example of a blood pressure measurement system.
FIGs. 2A-2C are diagrams illustrating a finger cuff according to optional examples.
FIGs. 3A-3B are diagrams illustrating light pipes within a finger cuff according to optional examples.
FIG. 4 is a diagram illustrating a finger cuff having a flexible circuit according to optional examples.
FIGs. 5A-5B are diagrams illustrating another finger cuff according to optional examples.
FIG. 6 is a block diagram illustrating an example environment in which optional examples may be practiced.

### DETAILED DESCRIPTION

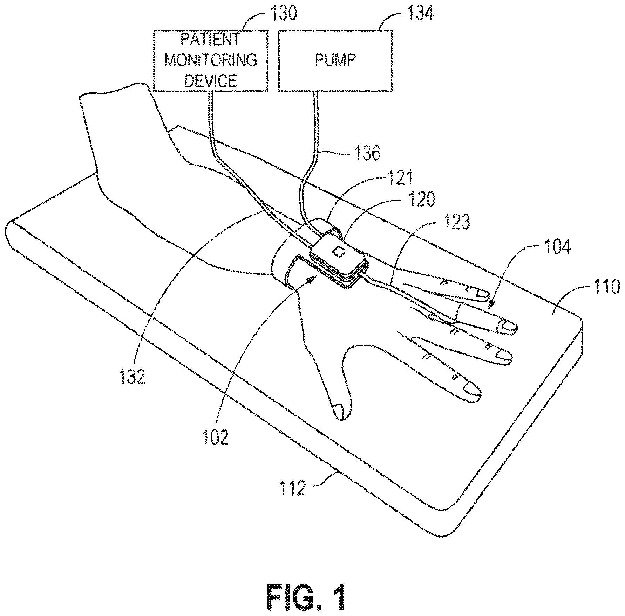

With reference to Figure 1, which illustrates an optional example of a blood pressure measurement system, a blood pressure measurement system 102 that includes a finger cuff 104 that may be attached to a patient's finger and a blood pressure measurement controller 120, which may be attached to the patient's body (e.g., a patient's wrist or hand) is shown.

The blood pressure measurement system 102 may further be connected to a patient monitoring device 130, and, in some optional examples, a pump 134. Further, finger cuff 104 includes a bladder (not shown) and an LED-PD pair (not shown), which are conventional for finger cuffs.

In one optional example, the blood pressure measurement system 102 may include a pressure measurement controller 120 that includes: a small internal pump, a small internal valve, a pressure sensor, and control circuitry. In this optional example, the control circuitry may be configured to: control the pneumatic pressure applied by the internal pump to the bladder of the finger cuff 104 to replicate the patient's blood pressure based upon measuring the volume or plethysmogram (pleth) signal received from the LED-PD pair of the finger cuff 104 (e.g., to keep the pleth signal constant). Further, the control circuitry may be configured to: control the opening of the internal valve to increase and release pneumatic pressure from the bladder; or the internal valve may simply be an orifice that is not controlled. Additionally, the control circuitry may be configured to: measure the patient's blood pressure by monitoring the pressure of the bladder based upon the input from a pressure sensor, which should be the same as patient's blood pressure, and may display the patient's blood pressure on the patient monitoring device 130.

In another optional example, a conventional pressure generating and regulating system may be utilized, in which, a pump 134 is located remotely from the body of the patient. In this optional example, the blood pressure measurement controller 120 receives pneumatic pressure from remote pump 134 through tube 136 and passes on the pneumatic pressure through tube 123 to the bladder of finger cuff 104. Blood pressure measurement device controller 120 may also control the pneumatic pressure (e.g., utilizing a controllable valve) applied to the finger cuff 104, as well as other functions. In this optional example, the pneumatic pressure applied by the pump 134 to the bladder of finger cuff 104 to replicate the patient's blood pressure based upon measuring the pleth signal received from the LED-PD pair of the finger cuff 104 (e.g., to keep the pleth signal constant) and measuring the patient's blood pressure by monitoring the pressure of the bladder may be controlled by the blood pressure measurement controller 120 and/or a remote computing device and/or the pump 134 and/or the patient monitoring device 130 to implement the volume clamping method. In some optional examples, a blood pressure measurement controller 120 is not used at all and there is simply a connection from tube 136 from a remote pump 134 including a remote pressure regulatory system to finger cuff 104, and all processing for the pressure generating and regulatory system, data processing, and display is performed by a remote computing device.

Continuing with this optional example, as shown in Figure 1, a patient's hand may be placed on the face 110 of an arm rest 112 for measuring a patient's blood pressure with the blood pressure measurement system 102. The blood pressure measurement controller 120 of the blood pressure measurement system 102 may be coupled to a bladder of the finger cuff 104 in order to provide pneumatic pressure to the bladder for use in blood pressure measurement. Blood pressure measurement controller 120 may be coupled to the patient monitoring device 130 through a power/data cable 132. Also, in one optional example, as previously described, in a remote implementation, blood pressure measurement controller 120 may be coupled to a remote pump 134 through tube 136 to receive pneumatic pressure for the bladder of the finger cuff 104. The patient monitoring device 130 may be any type of medical electronic device that may read, collect, process, display, etc., physiological readings/data of a patient including blood pressure, as well as any other suitable physiological patient readings. Accordingly, power/data cable 132 may transmit data to and from patient monitoring device 130 and also may provide power from the patient monitoring device 130 to the blood pressure measurement controller 120 and finger cuff 104.

As can be seen in Figure 1, the finger cuff 104 is attached to a patient's finger and the blood pressure measurement controller 120 may be attached on the patient's hand or wrist with an attachment bracelet 121 that wraps around the patient's wrist or hand. The attachment bracelet 121 may be metal, plastic, Velcro, etc. It should be appreciated that this is just one example of attaching a blood pressure measurement controller 120 and that any suitable way of attaching a blood pressure measurement controller to a patient's body or in close proximity to a patient's body may be utilized and that, in some optional examples, a blood pressure measurement controller 120 may not be used at all. It should further be appreciated that the finger cuff 104 may be connected to a blood pressure measurement controller described herein, or a pressure generating and regulating system of any other kind, such as a pressure generating and regulating system that is located remotely from the body of the patient. Any kind of pressure generating and regulating system can be used, including but not limited to the blood pressure measurement controller, and may be described simply as a pressure generating and regulating system that may be used with a finger cuff 104 including an LED-PD pair and a bladder to implement the volume clamping method.

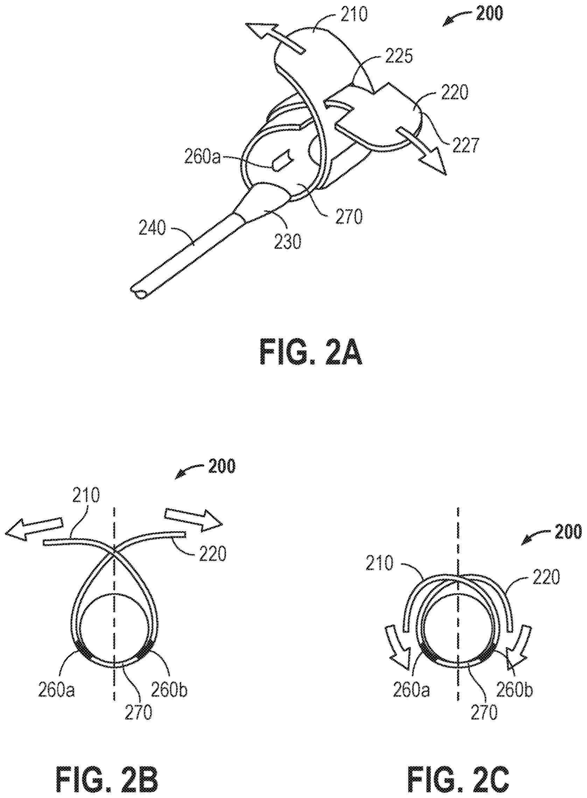

With reference to FIGs. 2A-2C, a finger cuff 200 will be particularly described. The finger cuff 200 may be the finger cuff 104, as previously described in FIG. 1. As shown, finger cuff 200 wraps around a patient's finger. The finger cuff 200 may be of flexible material with one or more fastening systems (e.g., a Velcro type component). As shown in FIGs. 2A-2C, according to the invention, finger cuff 200 includes a first end 210 and a second end 220. In the invention, the first end 210 includes a slot 225 and the second end 220 includes a portion 227, which may be a U-shaped portion. For attachment purposes to the patient's finger, the second end 220 (along with the portion 227) is pulled towards the first end 210, for example by a healthcare provider, and inserted or slid through the slot 225 to form a butterfly-shaped finger cuff (e.g., butterfly flaps from first end 210 and second end 220), to wrap or attach finger cuff 200 around the patient's finger. In some optional examples, the width of portion 227 may be larger than the slot 225 to prevent the second end 220 (and portion 227) from sliding back out after it is inserted through the slot 225.

With reference to FIG. 2B, after sliding the portion 227 through the slot 225, the first end 210 and second end 220 are pulled away from one another to a rotational position in order to apply a correct or desired tightness to the patient's finger. In applying the desired tightness to the patient's finger, the finger cuff 200 may include a built-in range limitation, for example provided by the amount of slack in the first end 210 and second end 220, that indicates whether the patient's finger is suitable (e.g., too small, too large, etc.) for the finger cuff 200. The built-in range limitation of finger cuff 200 may further provide an indication of correctness (e.g., correct or incorrect) with respect to the positioning of the finger cuff 200 on the patient's finger. As an example, the inability to apply certain tightness to the patient's finger using the finger cuff 200 may provide an intuitive feedback that the finger cuff 200 is too large or too small for the patient's finger. If the finger cuff 200 is not properly fitted, a differently sized finger cuff 200 (e.g., small, medium, larger, extra-large, etc.) may be selected and utilized instead.

With reference to FIG. 2C, when the desired tightness of the finger cuff 200 is obtained, the first end 210 and second end 220 are fastened to the exterior of the finger cuff 200 to maintain the desired tightness on the patient's finger. According to the invention, the first end 210 on the interior includes a fastening component (e.g., a Velcro type component) that connects with another fastening component (e.g., a Velcro type component) on the exterior of the finger cuff 200. Similarly, the second end 220 on the interior also includes a fastening component (e.g., a Velcro type component) that connects with the fastening component on the exterior of the finger cuff 200. In another optional example, the fastening components of the first end 210 and second end 220 may include removable or reusable adhesive material that may be fixedly or removably attached to the exterior surface of the finger cuff 200. It should be appreciated that these are just some examples of a fastening mechanism and that any suitable type may be utilized. In various optional examples, the butterfly flaps from first end 210 and second end 220 of finger cuff 200 may facilitate the pulling on both ends of the finger cuff 200, thereby facilitating a healthcare provider, for example, to apply a correct tightness to the patient's finger, apply a correct rotational positioning and obtain an accurate blood pressure measurement. In addition, the built-in range limitation of finger cuff 200 may automatically prevent placing a finger cuff that is inadequate (e.g., too small or too large) for a certain finger size.

As further shown in FIGs. 2A-2C, finger cuff 200 includes a bladder 270 and an LED-PD pair 260a-b mounted on the interior of the finger cuff 200. In one alternative of the invention, the bladder 270 includes a pair of openings that surround the LED-PD pair 260a-b. The bladder 270 and LED-PD pair 260a-b may couple to tube or cable 240 through a fixed connector 230, which may be attached to finger cuff 200, to provide pneumatic pressure to the bladder 270, and to provide power to and receive data from the LED-PD pair 260a-b. It should be appreciated that the previously described butterfly-shaped finger cuff may be used independently or may be used in combination with one or more other optional examples to be hereafter described (e.g., light pipes optional example, flexible circuit optional example, multiple LEDs optional example, etc.).

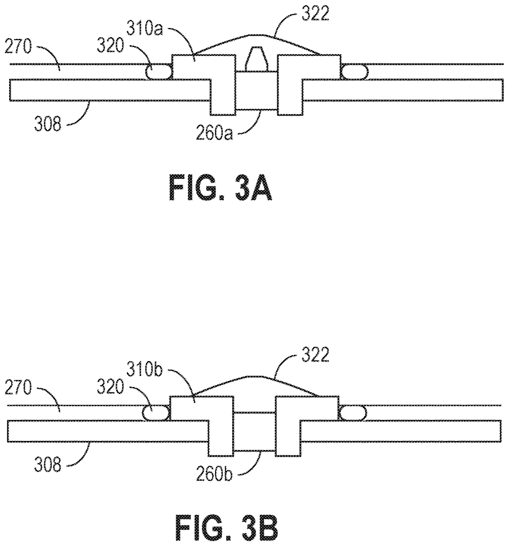

With reference to FIGs. 3A-3B, an optional example related to light pipes within a finger cuff will be particularly described. As previously described, finger cuff 200 includes bladder 270 having a pair of openings that surround the LED-PD pair 260a-b. However, in another alternative of the invention, openings may not be present in one or both layers of the bladder 270 (e.g., the bladder 270 is continuous) and the LED-PD pair 260a-b is simply under the bladder 270 or under one of the layers of the bladder 270. Since the bladder 270 is translucent, openings may not be required for optical transmission of light.

With reference to FIG. 3A, in one optional example, finger cuff 200 may also include a first light pipe 310a that cylindrically surrounds LED 260a and that is mounted to the backing layer 308 of the finger cuff 200, in which, the backing layer holds the bladder 270 on its inside and the LED 260a. Similarly, with reference to FIG. 3B, finger cuff 200 may also include a second light pipe 310b that cylindrically surrounds PD 260b and that is mounted to the backing layer 308 of the finger cuff 200, in which, the backing layer holds the bladder 270 on its inside and the PD 260b. As one optional example, the light pipes 310a and 310b may be approximately cup-shaped having a flat part and a vertical part. The flat parts extend away from the openings holding the LED 260a and PD 260b, respectively, and may abut the bladder 270, and in some optional examples, sealing edges 320 between the flat parts and bladder may be present, formed by the sealing process. Also, in some optional examples, an epoxy 322 may further seal the LED 260a and PD 260b to their respective light pipes 310a and 310b. The flat parts of the light pipes 310a and 310b may perform multiple functions, including: 1) the flat parts provide easier mounting of the LED 260a and PD 260b in the openings of the bladder 270 and backing layer 308; the flat parts in cooperation with the vertical parts operate as light pipes, as will be described in more detail hereafter; and 3) the flat parts provide a better coupling of light into the skin tissue because they constitute a flat and somewhat protruding surface for this interface, such that, the LED 260a and PD 260b protrude a bit (and therefore are not recessed, as is often the case), reducing the air gap - Any air gap between the LED and skin will generate a lot of stray light that is likely to travel around the finger (bouncing back and forth between the skin and cuff) and will not travel through it and see the artery, as is intended. The flat parts may be separate from the vertical cylindrical parts and may be referred to as guiding rings.

It should be appreciated that an objective of the light pipes 310a and 310b is to avoid stray light photons going sideways, and not going straight ahead, through the finger. Therefore, the light pipes can be me made either from absorbing material (take away stray photons), optically opaque material, or reflective material (re-routing and re-focusing stray photons). Also, an objective is to provide direct coupling, without an air gap, and without an LED or PD tilted over a certain angle, such that a positioning objective is also met. It should be appreciated that the light pipes 310a and 310b surrounding the LED and PD 206a and 260b, respectively, may serve to guide and focus light emitted from the LED 260a into a specific photon banana path extending from the LED 260a to the PD 260b and may effectively limit the photon banana width to an intended section of the finger arteries within the patient's finger in order to increase the accuracy in the blood pressure measurement. As previously described, the light from the LED 260a may travel along a specific photon banana path extending from the LED 260a to the PD 260b. In some optional examples, the light pipes 310a and 310b may be mounted underneath the bladder 270. In some optional examples, the light pipes 310a and 310b may be approximately cylindrically-shaped or of any suitable shape. In some optional examples, the light pipes 310a and 310b may be made from absorbing material, optically opaque material, reflective material, and/or flexible material. Although, an LED source is provided as the invention's embodiment of a light or optical source, it should be appreciated that any suitable LED source (red, blue, or alternative LED types) or any type of light source may utilized. As an optional example, a laser source utilizing a small bundle aperture could be used as a light source. It should be appreciated that the previously described light pipes optional example may be used independently or may be used in combination with one or more other optional examples previously and/or hereafter described (e.g., butterfly-shaped finger cuff, flexible circuit optional example, multiple LEDs optional example, etc.).

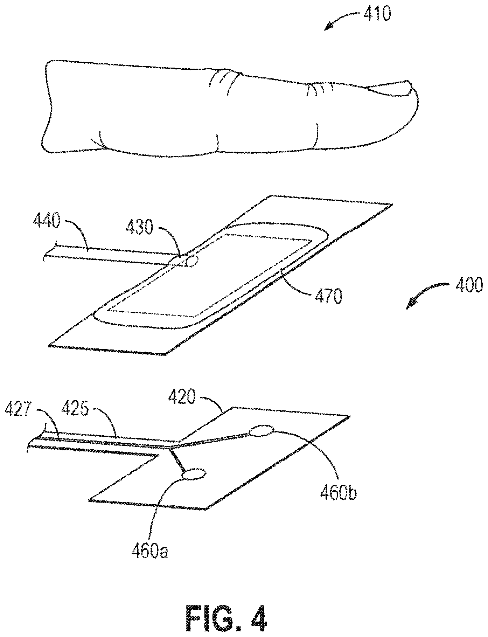

With reference to FIG. 4, optional examples related to a finger cuff 400 having a flexible circuit 420 will be particularly described. In some optional examples, the finger cuff 400 may be the finger cuff 200 of FIGs. 2A-2C. As shown, finger cuff 400 may wrap around a patient's finger 410. As illustrated in FIG. 4, finger cuff 400 may include the flexible (or flex) circuit 420 and an inflatable bladder 470 (which may be of flexible and elastic material, e.g., polyurethane). The flexible circuit 420 may be mounted on the interior of the finger cuff 400 (e.g., the wrappable portion) and the inflatable bladder 470 may be mounted over the flexible circuit 420 also onto the interior of the finger cuff (e.g., the dashed lines under the bladder 470 representing the flexible circuit 420). As illustrated in FIG. 4, flexible circuit 420 may include a pair of openings 460a-b for accommodating an LED-PD pair (e.g., LED-PD pair 260a-b), which may be electrically connectable to flexible circuit 420. Alternatively, in one optional example, the LED-PD pair may be directly mounted on the flexible circuit 420. The flexible circuit 420 may include circuitry or electronic components (not shown) that process signals (e.g., pleth signals) from the LED-PD pair and communicate the signals to another component (e.g., control circuitry as discussed in more detail herein below). In some optional examples, flexible circuit 420 may be electrically connectable to a cable 425 via signal trace (or wire) 427 to provide power to and receive data from the LED-PD pair. In addition, bladder 470 may be coupled or connectable to tube 440 via connector 430 to provide pneumatic pressure to the bladder 470.

In one optional example, the flexible circuit 420 may be of flexible material (e.g., flexible polymer material). As can be seen in FIG. 4, the width of the flexible circuit 420 may be smaller than the full width of finger cuff 400. Further, the flexible circuit 420 may have soft and flexible edges to allow for adjustment to different finger characteristics (e.g., finger phalanx and knuckle anatomy) and may provide a tight fit and improved pressure transmission from the finger cuff 400 to the patient's finger. It should be appreciated that the flexible circuit 420 may be removably or fixedly attached to the interior of the finger cuff and similarly the bladder 470 may be removably or fixedly attached to the interior of the finger cuff. Also, in some optional examples, the flexible circuit 420 may be attached on top of the bladder 470. Accordingly, in some optional examples, flexible circuit 420 and/or bladder 470 may be physically separated or detached from one another and from the finger cuff 400.

As can be seen in FIG. 4, the flexible circuit 420 has a smaller width than the full width of the finger cuff. Further, the flexible circuit 420 may be formed to have soft, flexible edges to allow for certain adjusting to different finger phalanx and knuckle anatomy. Likewise, the inflatable bladder 470 may also have a smaller width in comparison to the full width of the finger cuff to similarly allow for certain adjusting to different finger phalanx and knuckle anatomy. Moreover, the flex circuit 420 and the inflatable bladder 470 may have reduced length in comparison to the interior of the finger cuff (e.g., the flex circuit 420 and the inflatable bladder 470 start later and end sooner than in present designs), which provide further benefits, as will be described. In particular, when the finger cuff 400 utilizes the butterfly design implementation, the more flexible material of the flex circuit 420, in combination with the butterfly design, makes it easier for a healthcare provider to obtain a good fit against the finger tissues (and therefore a correct pressure transmission from the finger cuff to the finger tissue), and ultimately to the outside of the arterial wall of the two finger arteries under the finger cuff, even on fingers with large knuckles. More particularly, flexible edges of the flex circuit 420 are applied to the patient, such that, traditional rigid edges of traditional circuits, do not sit on the patient's knuckles and cause blood vessel obstruction, nerve compression, and pain. The goodness of fit is especially crucial on the ventral side of the finger, since the two arteries are located at that side, under the bone and alongside the tendon

Further, by intentionally leaving a strip open (e.g., 5-10 mm - for a large cuff) on the dorsal side, which is achievable by the less lengthy and less wide inflatable bladder 470 and flex circuit 420, allows for (part of) the veins on the dorsal side of the finger to remain (more or less) open even during inflation of the bladder 470 to arterial pressure level. This can play a role in the prevention of blue finger tips and numbness in fingers during a prolonged measurement. It should be appreciated that the previously described flexible circuit optional example may be used independently or may be used in combination with one or more other optional examples previously and/or hereafter described (e.g., butterfly-shaped finger cuff, light pipes optional example, multiple LEDs optional example, etc.).

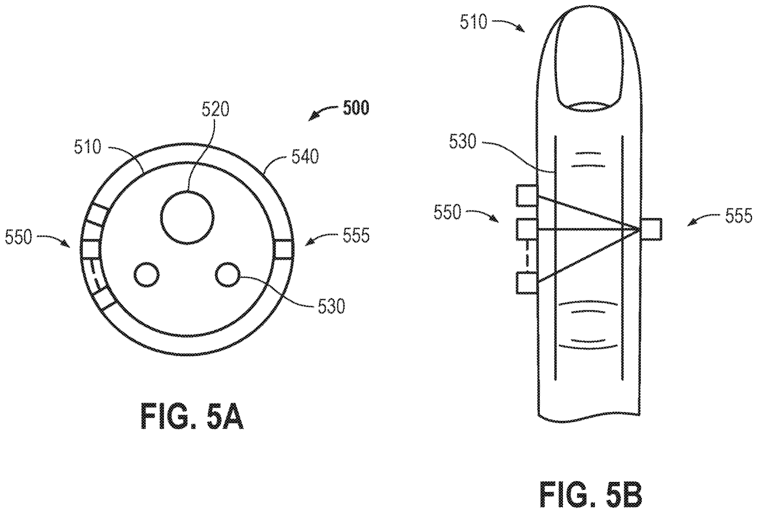

With reference to FIGs. 5A-5B, optional examples related to a finger cuff 500 with multiple LEDs will be particularly described. In some optional examples, finger cuff 500 may be the finger cuff 200 of FIG. 2A-2C or the finger cuff 400 of FIG. 4. As shown, finger cuff 500 may be wrapped around a finger 510 having finger bone 520 and finger arteries 530. In one optional example, the finger cuff 500 may include a bladder 540, two or more LEDs 550 and a PD 555 mounted on the interior of the finger cuff 500. In one optional example, the bladder 540 may include openings that surround the LEDs 550 and PD 555. The bladder 540, LEDs 550, and PD 555 may be coupled to a tube or cable (not shown) through a connector (also not shown), which may be attached to finger cuff 500, to provide pneumatic pressure to the bladder 540, to provide power to the LEDs 550 and to receive data from the PD 555.

Operationally, LEDs 550 may concurrently, alternatively, or in pre-defined sequences, transmit or emit light in different wavelengths and in different directions through finger arteries 530. In this scenario, the light from the LEDs 550 may be detected and registered by the PD 555 to generate a more accurate and optimal quality pleth signal, which may indicate an optimal location of the LED with respect to the location of finger arteries 530 within the patient's finger 510 for measuring the patient's blood pressure.

Further, by using more than one LED 550, additional measurements may be obtained (e.g., oxygen saturation and other physiological blood parameters, such as glucose) from signals provided by PD 555, and noise may be reduced (e.g., noise within oxygen saturation measurements).

By utilizing the previously described multiple LED 550 (two or more) volume clamp implementation, as described above, additional options are provided to measure Oxygen Saturation and other physiological blood parameter measurements during continuous blood pressure measurement, in a potentially more reliable way, as opposed to current procedures. In particular, by using the previously described multiple LED 550 volume clamp implementation, because measurements are made in a conduit artery 530, in the middle phalanx, as opposed to a capillary and arteriolar bed in the fingertip (as with current procedures), the impact of vasoconstriction (arteriolar) is reduced to a major extent. In particular, the measurement compartments can be controlled that contribute to absorption signal information, from all compartments (zero cuff pressure) to arteriolar + arterial compartment (low cuff pressure, veins collapsed) to only the arterial compartment (volume clamp at unloaded volume of arteries). In this way, much of the noise typically confounding a traditional Sp02 measurement can be taken away. In particular, during the volume clamp procedure, the blood flow in the arteries - another important confounder - is reduced to only a tiny inward and backward arterial flow, which also reduces noise compared to traditional Sp02 measurement. Further, during the volume clamp procedure, less problems exist with blood sloshing in the arteries because of motion artifacts. It should be noted that when the goal is to measure in the arterial compartment, such as the case in oxygen saturation measurements, any signal component related to tissue or arteriolar, capillary or venous compartments can be seen as noise. Also, the previously described optional examples combine a plurality of LEDs of different directions and wavelengths and possibly applying pressure by the bladder. Pressure in the bladder can be either constant or in a prescribed wave pattern - such as a sinus - or dynamically tracking the intra-arterial pressure as is the case during volume clamp. The purpose of the pressure thus may be two-fold: measure blood pressure and compress/collapse the compartments which may generate signal components that act as noise in the oxygen saturation measurement. Noise may be reduced as previously described utilizing a PD with multiple LEDs.

By utilizing the previously described multiple LED 550 volume clamp implementation, local oxygenation information can be measured from under the finger cuff, and this information can be used to guide an intelligent, physiology driven strategy for recommending a switch to another finger or a rest period. Therefore, this measurement system may be turned into an expert advising system based on actual information derived from the patient's local circumstances at that time. It should be appreciated that the previously described multiple LEDs optional example may be used independently or may be used in combination with one or more other optional examples previously and/or hereafter described (e.g., butterfly-shaped finger cuff optional example, light pipes optional example, flexible circuit optional example, etc.).

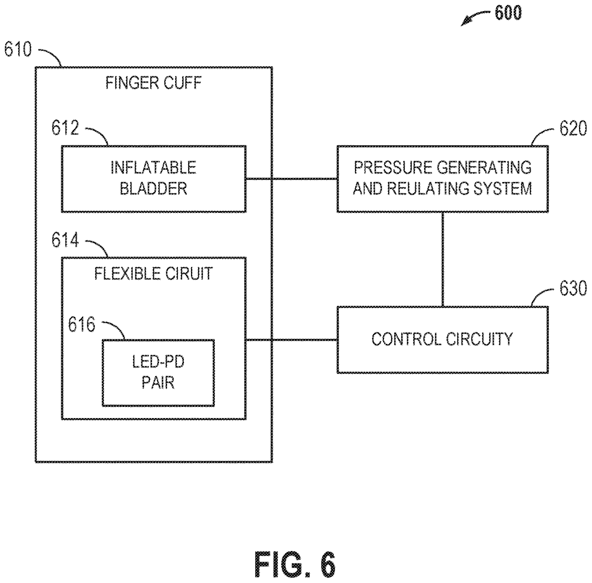

FIG. 6 is a block diagram illustrating an optional example environment 600 in which the optional examples may be practiced. As shown, finger cuff 610 may include an inflatable bladder 612 and a flexible circuit 614. The flexible circuit 614 may be coupled or connectable to the LED-PD pair 616 to process the signals (e.g., pleth signals) from the photodiode and communicate the signals to control circuitry 630. The inflatable bladder 612 may be pneumatically connected to a pressure generating and regulating system 620. The pressure generating and regulating system 620 may generate, measure, and regulate pneumatic pressure that inflates or deflates the bladder 612, and may include elements such as a pump, a valve, a sensor, control circuitry, and/or other suitable elements. In particular, pressure generating and regulating system 620 in cooperation with control circuitry 630 may be configured to implement a volume clamp method with the finger cuff 610 by: applying pneumatic pressure to the inflatable bladder 612 of the finger cuff 610 to replicate the patient's blood pressure based upon measuring pleth signals received from the flexible circuit 614 (e.g., to keep the pleth signal constant), and measuring the patient's blood pressure by monitoring the pressure of the inflatable bladder 612 based upon input from a pressure sensor, which should be the same or correlated to the patient's blood pressure, and may further command the display of the patient's blood pressure on the patient monitoring device.

It should be appreciated that the previously described butterfly-shaped finger cuff optional example, light pipes optional example, flexible circuit optional example, and multiple LEDs optional example may each be used independently or each may be used with one or more of the other optional examples.

It should be appreciated that aspects of the invention previously described may be implemented in conjunction with the execution of instructions by processors, circuitry, controllers, control circuitry, etc. As an example, control circuitry may operate under the control of a program, algorithm, routine, or the execution of instructions to execute methods or processes in accordance with embodiments of the invention previously described. For example, such a program may be implemented in firmware or software (e.g. stored in memory and/or other locations) and may be implemented by processors, control circuitry, and/or other circuitry, these terms being utilized interchangeably. Further, it should be appreciated that the terms processor, microprocessor, circuitry, control circuitry, circuit board, controller, microcontroller, etc., refer to any type of logic or circuitry capable of executing logic, commands, instructions, software, firmware, functionality, etc., which may be utilized to execute embodiments of the invention.

The various illustrative logical blocks, processors, modules, and circuitry described in connection with the embodiments disclosed herein may be implemented or performed with a general purpose processor, a specialized processor, circuitry, a microcontroller, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A processor may be a microprocessor or any conventional processor, controller, microcontroller, circuitry, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module/firmware executed by a processor, or any combination thereof. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such the processor can read information from, and write information to, the storage medium. In the alternative, the storage medium may be integral to the processor.

## Claims

1. A finger cuff (104, 200, 400, 500, 600) attachable to a patient's finger to be used in measuring the patient's blood pressure by a blood pressure measurement system (102), the finger cuff (104, 200, 400, 500, 600) comprising:
a first end (210) and a second end (220), wherein the first end (210) includes a slot (225) and the second end (220) includes a portion (227) that is configured to, when attaching the finger cuff to the patient's finger, slide through the slot (225) to form a butterfly shape in which the first end (210) and the second end (220) can be pulled away from one another to apply tightness when attached around the patient's finger, wherein the first end (210) and the second end (220) include a fastening component that fastens to the exterior of the finger cuff (104, 200, 400, 500, 600) in order to maintain a desired tightness on the patient's finger;
a light emitting diode (LED) - photodiode (PD) pair (260a-b, 550, 555, 616); and
a bladder (270, 470, 612), which
either includes a pair of openings, the bladder (270, 470) mounted within the finger cuff (104, 200, 400, 500) such that the pair of openings surround the LED-PD pair (260a-b), respectively,
or is continuous and translucent to accommodate the LED-PD pair in or under the bladder;
wherein the patient's finger surrounded in the finger cuff (104, 200, 400, 500) abuts against the bladder with the desired tightness maintained by the fastening component such that the bladder and the LED-PD pair are used in measuring the patient's blood pressure by the blood pressure measurement system (102).

2. The finger cuff (104, 200, 400, 500, 600) of claim 1, wherein the portion (227) is a U-shaped portion, wherein the width of the U-shaped portion (227) is larger than the slot (225) to prevent the U-shaped portion (227) from sliding out after the U-shaped portion (227) is slid through the slot (225), wherein
the U-shaped portion (227) slides through the slot to a rotational position to provide a built-in range limitation indicating whether the patient's finger is suitable for the finger cuff (104, 200, 400, 500, 600).

3. A method to measure a patient's blood pressure by a blood pressure measurement system (102) utilizing a finger cuff (104, 200, 400, 500, 600), the finger cuff (104, 200, 400, 500, 600) including a light emitting diode (LED) - photodiode (PD) pair (260a-b, 550, 555, 616) and a bladder (270, 470, 612), the method comprising:
placing the finger cuff around the patient's finger such that the bladder (270, 470, 612) and the LED-PD pair (260a-b, 550, 555, 616) aid in measuring the patient's blood pressure by the blood pressure measurement system (102), wherein
the finger cuff (104, 200, 400, 500, 600) includes a first end (210) and a second end (220), wherein the first end (210) includes a slot (225) and the second end (220) includes a portion (227), wherein the first end (210) and the second end (220) include a fastening component that fastens to the exterior of the finger cuff (104, 200, 400, 500, 600) in order to maintain the tightness on the patient's finger;
sliding the portion (227) of the second end (220) through the slot (225) of the first end (210), thereby forming a butterfly shape when attached around the patient's finger;
pulling the first end (210) and the second end (220) away from one another to apply tightness to the patient's finger, and
fastening the first end (210) and the second end (220) to the exterior of the finger cuff (104, 200, 400, 500, 600) using the fastening component to maintain the tightness on the patient's finger.

4. The method of claim 3, wherein the portion is a U-shaped portion and the width of the U-shaped portion (227) is larger than the slot (225) to prevent the U-shaped portion (227) from sliding out after the U-shaped portion (227) is slid through the slot (225), the step of sliding the second end (220) through the first end (210) to form the butterfly shape comprises sliding the U-shaped portion (227) through the slot (225) to a rotational position to provide a built-in range limitation indicating whether the patient's finger is suitable for the finger cuff (104, 200, 400, 500, 600).

## Patentansprüche

1. Fingermanschette (104, 200, 400, 500, 600), die an einem Finger eines Patienten anbringbar ist, um zur Messung des Blutdrucks des Patienten durch ein Blutdruckmesssystem (102) verwendet zu werden, wobei die Fingermanschette (104, 200, 400, 500, 600) umfasst:
ein erstes Ende (210) und ein zweites Ende (220), wobei das erste Ende (210) einen Schlitz (225) einschließt, und das zweite Ende (220) einen Abschnitt (227) einschließt, der ausgestaltet ist, um, wenn die Fingermanschette am Finger des Patienten angebracht wird, durch den Schlitz (225) zu gleiten, um eine Schmetterlingsform zu bilden, bei der das erste Ende (210) und das zweite Ende (220) voneinander weg gezogen werden können, um Straffheit anzuwenden, wenn sie um den Finger des Patienten herum angebracht sind, wobei das erste Ende (210) und das zweite Ende (220) eine Befestigungskomponente einschließen, die an dem Äußeren der Fingermanschette (104, 200, 400, 500, 600) befestigt ist, um eine gewünschte Straffheit am Finger des Patienten aufrechtzuerhalten;
ein Paar aus lichtemittierender Diode (LED) - Photodiode (PD) (260a-b, 550, 555, 616); und
eine Blase (270, 470, 612), die
entweder ein Paar Öffnungen einschließt, wobei die Blase (270, 470) innerhalb der Fingermanschette (104, 200, 400, 500) montiert ist, so dass das Paar der Öffnungen jeweils das LED-PD-Paar (260a-b) umgibt,
oder kontinuierlich und durchscheinend ist, um das LED-PD-Paar in oder unter der Blase unterzubringen;
wobei der Finger des Patienten, der in der Fingermanschette (104, 200, 400, 500) umgeben ist, an der Blase anliegt, wobei die gewünschte Straffheit durch die Befestigungskomponente aufrechterhalten wird, so dass die Blase und das LED-PD-Paar zur Messung des Blutdrucks des Patienten durch das Blutdruckmesssystem (102) verwendet werden.

2. Fingermanschette (104, 200, 400, 500, 600) nach Anspruch 1, wobei der Abschnitt (227) ein U-förmiger Abschnitt ist, wobei die Breite des U-förmigen Abschnitts (227) größer als der Schlitz (225) ist, um zu verhindern, dass der U-förmige Abschnitt (227) herausrutscht, nachdem der U-förmige Abschnitt (227) durch den Schlitz (225) gleiten gelassen wurde, wobei
der U-förmige Abschnitt (227) durch den Schlitz hindurch in eine Rotationsposition gleitet, um eine eingebaute Bereichsgrenze bereitzustellen, die angibt, ob der Finger des Patienten für die Fingermanschette (104, 200, 400, 500, 600) geeignet ist.

3. Verfahren zum Messen des Blutdrucks eines Patienten durch ein Blutdruckmesssystem (102) unter Verwendung einer Fingermanschette (104, 200, 400, 500, 600), wobei die Fingermanschette (104, 200, 400, 500, 600) ein Paar aus lichtemittierender Diode (LED) - Photodiode (PD) (260a-b, 550, 555, 616) und eine Blase (270, 470, 612) einschließt, wobei das Verfahren umfasst:
Platzieren der Fingermanschette um den Finger des Patienten herum, so dass die Blase (270, 470, 612) und das LED-PD-Paar (260a-b, 550, 555, 616) das Messen des Blutdrucks des Patienten durch das Blutdruckmesssystem (102) unterstützen, wobei
die Fingermanschette (104, 200, 400, 500, 600) ein erstes Ende (210) und ein zweites Ende (220) einschließt, wobei das erste Ende (210) einen Schlitz (225) einschließt und das zweite Ende (220) einen Abschnitt (227) einschließt, wobei das erste Ende (210) und das zweite Ende (220) eine Befestigungskomponente einschließen, die an dem Äußeren der Fingermanschette (104, 200, 400, 500, 600) befestigt wird, um die Straffheit am Finger des Patienten aufrechtzuerhalten;
Gleitenlassen des Abschnitts (227) des zweiten Endes (220) durch den Schlitz (225) des ersten Endes (210), wodurch eine Schmetterlingsform gebildet wird, wenn die Anbringung um den Finger des Patienten herum erfolgt;
Ziehen des ersten Endes (210) und des zweiten Endes (220) voneinander weg, um Straffheit auf den Finger des Patienten anzuwenden, und
Befestigen des ersten Endes (210) und des zweiten Endes (220) an dem Äußeren der Fingermanschette (104, 200, 400, 500, 600) unter Verwendung der Befestigungskomponente, um die Straffheit an dem Finger des Patienten aufrechtzuerhalten.

4. Verfahren nach Anspruch 3, wobei der Abschnitt ein U-förmiger Abschnitt ist und die Breite des U-förmigen Abschnitts (227) größer als der Schlitz (225) ist, um zu verhindern, dass der U-förmige Abschnitt (227) herausrutscht, nachdem der U-förmige Abschnitt (227) durch den Schlitz (225) gleiten gelassen wird, wobei der Schritt des Gleitenlassens des zweiten Endes (220) durch das erste Ende (210) hindurch, um die Schmetterlingsform zu bilden, Gleitenlassen des U-förmigen Abschnitts (227) durch den Schlitz (225) hindurch bis zu einer Rotationsposition umfasst, um eine eingebaute Bereichsgrenze bereitzustellen, die angibt, ob der Finger des Patienten für die Fingermanschette (104, 200, 400, 500, 600) geeignet ist.

## Revendications

1. Doigtier (104, 200, 400, 500, 600) pouvant être fixé au doigt d'un patient pour être utilisé dans la mesure de la tension artérielle du patient par un système de mesure de tension artérielle (102), le doigtier (104, 200, 400, 500, 600) comprenant :
une première extrémité (210) et une seconde extrémité (220), la première extrémité (210) comprenant une fente (225) et la seconde extrémité (220) comprenant une partie (227) qui est conçue pour, lors de la fixation du doigtier au doigt du patient, coulisser à travers la fente (225) afin de former une forme de papillon dans laquelle la première extrémité (210) et la seconde extrémité (220) peuvent être écartées l'une de l'autre afin d'appliquer une étanchéité lors de la fixation autour du doigt du patient, la première extrémité (210) et la seconde extrémité (220) comprenant un composant de fixation qui se fixe à l'extérieur du doigtier (104, 200, 400, 500, 600) afin de maintenir une étanchéité souhaitée sur le doigt du patient ;
une paire diode électroluminescente (DEL) - photodiode (PD) (260a-b, 550, 555, 616) ; et
une poche (270, 470, 612), qui
soit comprend une paire d'ouvertures, la poche (270, 470) étant montée à l'intérieur du doigtier (104, 200, 400, 500) de telle sorte que la paire d'ouvertures entoure respectivement la paire DEL-PD (260a-b),
soit est continue et translucide pour recevoir la paire DEL-PD dans ou sous la poche ;
le doigt du patient entouré dans le doigtier (104, 200, 400, 500) venant en butée contre la poche avec l'étanchéité souhaitée maintenue par le composant de fixation de telle sorte que la poche et la paire DEL-PD sont utilisées pour mesurer la tension artérielle du patient par le système de mesure de tension artérielle (102).

2. Doigtier (104, 200, 400, 500, 600) selon la revendication 1, la partie (227) étant une partie en forme de U, la largeur de la partie en forme de U (227) étant plus grande que la fente (225) pour empêcher la partie en forme de U (227) de sortir par coulissement après que la partie en forme de U (227) est coulissée à travers la fente (225),
la partie en forme de U (227) coulissant à travers la fente vers une position de rotation pour fournir une limitation de plage intégrée indiquant si le doigt du patient est approprié pour le doigtier (104, 200, 400, 500, 600) ou non.

3. Procédé destiné à mesurer la tension artérielle d'un patient par un système de mesure de tension artérielle (102) à l'aide d'un doigtier (104, 200, 400, 500, 600), le doigtier (104, 200, 400, 500, 600) comprenant une paire diode électroluminescente (DEL) - photodiode (PD) (260a-b, 550, 555, 616) et une poche (270, 470, 612), le procédé comprenant :
le placement du doigtier autour du doigt du patient de telle sorte que la poche (270, 470, 612) et la paire DEL-PD (260a-b, 550, 555, 616) aident à mesurer la tension artérielle du patient par le système de mesure de tension artérielle (102),
le doigtier (104, 200, 400, 500, 600) comprenant une première extrémité (210) et une seconde extrémité (220), la première extrémité (210) comprenant une fente (225) et la seconde extrémité (220) comprenant une partie (227), la première extrémité (210) et la seconde extrémité (220) comprenant un composant de fixation qui se fixe à l'extérieur du doigtier (104, 200, 400, 500, 600) afin de maintenir l'étanchéité sur le doigt du patient ;
le coulissement de la partie (227) de la seconde extrémité (220) à travers la fente (225) de la première extrémité (210), formant ainsi une forme de papillon lors de la fixation autour du doigt du patient ;
l'écartement de la première extrémité (210) et de la seconde extrémité (220) l'une de l'autre pour appliquer une étanchéité au doigt du patient et
la fixation de la première extrémité (210) et de la seconde extrémité (220) à l'extérieur du doigtier (104, 200, 400, 500, 600) à l'aide du composant de fixation afin de maintenir l'étanchéité sur le doigt du patient.

4. Procédé selon la revendication 3, la partie étant une partie en forme de U et la largeur de la partie en forme de U (227) étant plus grande que la fente (225) pour empêcher la partie en forme de U (227) de sortir par coulissement après que la partie en forme de U (227) est coulissée à travers la fente (225), l'étape de coulissement de la seconde extrémité (220) à travers la première extrémité (210) pour former la forme de papillon comprenant le coulissement de la partie en forme de U (227) à travers la fente (225) vers une position de rotation pour fournir une limitation de plage intégrée indiquant si le doigt du patient est approprié pour le doigtier (104, 200, 400, 500, 600) ou non.
